(19) Europäisches Patentamt / European Patent Office / Office européen des brevets

(11) **EP 1 250 918 B1**

(12) **FASCICULE DE BREVET EUROPEEN**

(45) Date de publication et mention
de la délivrance du brevet:
**14.02.2007 Bulletin 2007/07**

(51) Int Cl.:
*A61K 8/64* (2006.01)     *A61Q 17/04* (2006.01)
*A61Q 19/08* (2006.01)

(21) Numéro de dépôt: **02290986.5**

(22) Date de dépôt: **18.04.2002**

(54) **Compositions cosmétiques contenant des extraits d'archaebactéries**

Kosmetische Mittel, die Archaebakterienextrakte enthalten

Cosmetic compositions containing archaebacteria extracts

(84) Etats contractants désignés:
**AT BE CH CY DE DK ES FI FR GB GR IE IT LI LU MC NL PT SE TR**

(30) Priorité: **19.04.2001 FR 0105287**

(43) Date de publication de la demande:
**23.10.2002 Bulletin 2002/43**

(73) Titulaire: **CASTER**
**75008 Paris (FR)**

(72) Inventeur: **Rodelet, Jean-François**
**92100 Boulogne Billancourt (FR)**

(74) Mandataire: **Casalonga, Axel**
**Bureau Casalonga & Josse**
**Bayerstrasse 71/73**
**80335 München (DE)**

(56) Documents cités:
**EP-A- 0 286 869          EP-A- 0 461 662**
**FR-A- 2 590 273**

- **DATABASE CAPLUS [en ligne] CHEMICAL ABSTRACTS SERVICE, COLUMBUS, OHIO, US; retrieved from STN Database accession no. 1996: 441289 XP002192958**
- **DATABASE BIOSIS [en ligne] BIOSCIENCES INFORMATION SERVICE, PHILADELPHIA, PA, US; retrieved from STN Database accession no. 1999:323068 XP002192959**
- **[Online] extrait de WWW.FAD.BF.REFER.ORG**

EP 1 250 918 B1

**Description**

L'invention concerne l'utilisation d'une fraction glycoprotéique extraite d'une archaebactérie : *Halobacterium halobium*. Le produit de l'invention, incorporé à une préparation cosmétique, présente en effet la particularité de protéger les cellules de la peau des effets nocifs de la pollution et/ou des ultraviolets.

Les halobactéries sont classifiées dans le règne des archaebactéries, une des trois branches principales de l'arbre phylogénétique. Les deux autres sont les eubactéries (également appelées procaryotes) et les eucaryotes.

Les archaebactéries ont été identifiées dans toutes les niches extrêmes aux frontières de la vie : températures dépassant les 100°C, acidité à pH=0 ou concentration de sel pouvant dépasser les 30 %.

Les archaebactéries sont dotées de parois cellulaires atypiques dans lesquelles fait défaut un composant de la molécule de peptidoglycane, classique chez les bactéries, l'acide muramique. De plus, alors que les autres organismes fabriquent les lipides de leur membrane en assemblant deux chaînes d'acides gras avec une molécule de glycérol par l'intermédiaire d'une liaison ester, les lipides des archaebactéries sont composés de longues chaînes d'alcool isoprénique attachées au glycérol par des liaisons éther.

Les halobactéries sont des bactéries extrêmes obligatoires. Elles exigent en effet pour leur croissance de très fortes concentrations en sel (de 10 à 30 %), KCl, $MgCl_2$ et surtout NaCl. Ces organismes ont été isolés à partir de milieux naturels (Grand Lac Salé aux USA ou La Mer Morte en Israël) ou artificiels (marais salants). Pour le maintien de leur pression osmotique interne qui doit équilibrer la concentration de NaCl dans le milieu, les halobactéries accumulent de 3 à 4 M de sel dans leur cytoplasme sous forme de KCl. Une suspension de halobactéries dans un milieu à une concentration de 2M en NaCl entraîne la perte complète de la rigidité de l'enveloppe bactérienne et la bactérie prend alors une forme arrondie. Descendre la concentration en sel sous les 1M conduit à la lyse bactérienne.

Les colonies des halobactéries sont de couleur rouge, leurs enveloppes contiennent en effet des pigments colorés (les bactériorubérines) qui les protègent contre l'intense rayonnement ultraviolet auquel elles sont exposées.

Parmi les halobactéries, *Halobacterium halobium* possède de plus une enveloppe externe supplémentaire, la membrane pourpre, qui sert de support à un mécanisme photosynthétique original.

La forme classique de Halobacterium en milieu riche en sel est celle d'un bacille allongé de 4 à 10 $\mu$m de long et de 0,7 $\mu$m de diamètre. Cette bactérie possède de 5 à 8 flagelles lophotriches. *Halobacterium halobium* est incapable d'utiliser les carbohydrates comme source de carbone et d'énergie.

La faculté de résistance et les particularités de ces bactéries en font des outils très prometteurs pour l'industrie. Elles sont d'ailleurs déjà exploitées dans des secteurs aussi divers que l'agroalimentaire, le papier, les lessives ou l'industrie pharmaceutique.

La demande de brevet américain US 5 091 364 fait référence à la préparation de glycoprotéines d'enveloppe extraites de cultures d'archaebacteries dans le but, après dégradation enzymatique des glycoprotéines, de les utiliser pour augmenter les défenses immunitaires du corps contre l'infection.

La demande de brevet FR 2 590 273 utilise également des fractions issues des archaebactéries mais en association avec de l'eau de mer synthétique dans le cadre de la fabrication de produits esthétiques en dermatologie.

La demanderesse a découvert que la fraction glycoprotéique issue d'un culot bactérien de *Halobacterium halobium* avait des propriétés intéressantes en cosmétique notamment au niveau de la protection des cellules de la peau contre l'action nocive de la pollution par les gaz d'échappement et/ou des rayonnements ultraviolets.

L'invention a donc pour objet.

Un autre objet est constitué par des compositions cosmétiques contenant une fraction glycoprotéique extraite des archaebactéries.

D'autres objets apparaîtront à la lecture de la description et des exemples qui suivent.

Le produit objet de l'invention est caractérisé par le fait qu'il comprend une fraction glycoprotéique extraite d'archaebactéries.

Le produit selon l'invention comprend de 25 à 40 % de glycoprotéines d'archaebactéries.

De préférence, les archabactéries sont des halobactéries.

Le produit peut être obtenu de la façon suivante : la masse bactérienne issue de la culture d'archaebactéries est d'abord débarrassée de ses constituants lipidiques par deux extractions successives, la première avec un solvant halogéné et la seconde avec un alcanol en $C_1$-$C_4$, puis extraite par de l'eau distillée. L'extrait obtenu est ensuite ultra filtré afin d'éliminer les sels minéraux résiduels. Après évaporation et séchage sous vide du filtrat, on obtient une poudre blanc-jaune présentant une forte réaction positive à la ninhydrine.

Le procédé d'extraction du produit selon l'invention est appliqué à des archaebactéries, préférentiellement à des halobactéries et plus particulièrement à *Halobacterium halobium*.

La fraction glycoprotéique obtenue selon le procédé d'extraction de l'invention se caractérise par le fait qu'elle comprend de 25 à 40 % d'extrait glycoprotéique d'archaebactéries par gramme de fraction. Cette fraction a été dénommée SURVIUM.

Un autre objet de l'invention est l'utilisation de la fraction protéique de l'invention pour la préparation d'une formulation cosmétique pour la protection de la peau vis-à-vis de la pollution par les gaz d'échappement et/ou des rayonnements ultraviolets.

Les compositions cosmétiques de la présente invention sont caractérisées par le fait qu'elles comprennent, dans un milieu cosmétiquement acceptable une fraction glycoprotéique selon l'invention extraite d'archaebactéries et préférentiellement de *Halobacterium halobium*.

Les compositions cosmétiques de la présente invention peuvent contenir, en plus de la fraction glycoprotéique, de l'eau et des additifs habituellement utilisés en cosmétique. Ces additifs sont par exemple des agents épaississants, des parfums, des conservateurs, des émulsifiants, des huiles végétales ou minérales, des agents antiseptiques, des agents acidifiants ou alcalinisants, des vitamines, des agents anti-UV, des agents tensioactifs, des solvants, des agents stabilisateurs de pH, des silicones, etc.

Les compositions conformes à l'invention peuvent se présenter sous forme de lait, de crème, de lotion, de sérum, de masque, ou de gel.

Les exemples suivants sont destinés à illustrer l'invention sans toutefois présenter un caractère limitatif.

## <u>Exemple 1</u> : extraction de la fraction glycoprotéique issue de *Halobacterium halobium*

La masse bactérienne cultivée selon le protocole publié par D. Oesterhelt et W. Stoeckenius et congelée est fournie par le Centre National de la Recherche Scientifique sous la forme d'une substance compacte, granuleuse de couleur rougeâtre.

Une quantité de masse bactérienne est préalablement dispersée dans une petite partie de dichlorométhane (solvant non polaire) avec un appareil ultra-thurax à raison de 40 grammes de masse bactérienne pour 100 mL de solvant. Cette dispersion est versée dans une cartouche d'extraction en cellulose et le tout est placé dans le réceptacle de l'extracteur Soxhlet (Macherey-Nagel). Le ballon est rempli de 500 mL de solvant et l'appareil fermé est placé dans un bain-marie thermostaté à 85°C. Au bout de 6 heures d'extraction, la cartouche égouttée et séchée sous vide subit une deuxième extraction avec de l'éthanol à 99,9 % à une température de 90°C. Une dernière extraction est effectuée à l'eau distillée pendant 6 heures. La solution obtenue est ensuite concentrée dans un évaporateur rotatif sous vide (Rotavapor).

Le concentré obtenu contient une forte proportion de sels minéraux et doit être purifié. Il est redissous dans une quantité minimale d'eau distillée et la fraction saline est éliminée par ultrafiltration par utilisation d'une cellule Macrosep (Pall-Filtron), centrifugée à 5000 trs/min ce qui permet d'éliminer les constituants de masse moléculaire inférieurs à 1kD. La fraction purifiée est alors séchée dans un dessicateur sous vide. Le rendement d'extraction calculé est de 8 % de la masse bactérienne initiale.

## <u>Exemple 2</u> : Evaluation de la cytotoxicité de la fraction protéique à tester

La viabilité des tapis cellulaires est estimée par un test MTT. La quantification de l'activité métabolique des déshydrogénases mitochondriales se fait par mesure d'hydrolyse du MTT. En effet, la transformation de sel de tétrazolium incolore (MTT) en cristaux bleus de formazan est proportionnelle à l'activité d'une enzyme mitochondriale : la succinate déshydrogénase. Ainsi, la concentration de formazan est proportionnelle à la quantité de cellules vivantes dans le puits.

Le produit bactérien à tester, issu de la fraction glycoprotéique extraite, est mis en solution.

La solution stock est ainsi réalisée par dissolution de 20 % (poids/volume) du produit bactérien en milieu de culture.

Le test est réalisé sur cultures de kératinocytes humains. Les cellules sont cultivées en plaques 96 puits, en présence du produit à tester pendant 24 heures. Après ces 24 heures de contact, les cellules sont rincées et le milieu de culture est remplacé par du milieu contenant le MTT. Les cellules sont ensuite lysées et les cristaux de formazan sont solubilisés dans l'isopropanol acide. La quantité de formazan obtenue est quantifiée au spectrophotomètre à la longueur d'onde de 540nm.

La viabilité cellulaire (moyenne de trois essais) est calculée selon la formule

$$\% \text{ viabilité cellulaire} = (DO_{\text{cellules + produit}} / DO_{\text{cellules non traitées}}) \times 100$$

<u>Viabilité cellulaire en fonction de la quantité de produit à tester</u>

| % de la solution stock dans le milieu | Viabilité cellulaire en % |
|---|---|
| 0 | 100 |
| 0,00013 | 96 |
| 0,0006 | 109 |
| 0,0032 | 100 |
| 0,016 | 88 |
| 0,08 | 74 |
| 0,4 | 66 |
| 2 | 70 |
| 10 | 85 |

La concentration de produit bactérien qui est retenue pour les essais suivants est la dernière qui ne montre pas de toxicité vis-à-vis des cellules en culture. Ainsi, la concentration retenue est de 0,0032 % de la solution stock ce qui correspond à 6,4 $\mu$g/mL de produit.

### Exemple 3 : déterminations de la cytotoxicité du polluant utilisé

Le polluant est constitué de résidus de gaz d'échappement. Les particules de polluant sont récupérées sur des filtres. Les composants hydrosolubles de polluant sont d'abord élués en milieu de culture. Les fibres sont ensuite rincées à l'éthanol puis immergées dans du milieu et l'ensemble est vortexé. Après trois heures à température ambiante, la préparation est centrifugée à 1500 trs/min pendant 5 minutes. Le volume du surnageant est ajusté à 20 mL. Le milieu ainsi obtenu est alors homogène et de couleur grise.

La viabilité des tapis cellulaires est estimée par un test MTT décrit précédemment.

Viabilité des cellules en présence de polluant à différentes concentrations dans le milieu de culture

| Concentration en milieu pollué (en %) | Viabilité des cellules en % |
|---|---|
| 0 | 100 |
| 0,78 | 94 |
| 1,56 | 103 |
| 3,12 | 113 |
| 6,25 | 111 |
| 12,5 | 102 |
| 25 | 99 |
| 50 | 74 |
| 100 | 53 |

Dans les expériences suivantes, le polluant sera utilisé soit pur soit dilué à moitié.

### Exemple 4 : Quantification *in vitro* de l'effet du produit bactérien sur le métabolisme énergétique des kératinocytes humains en culture.

Le dosage d'ATP par méthode indirecte repose sur la mesure par bioluminescence des photons émis au cours de la réaction entre l'ATP et la D-luciférine en présence d'oxygène, de magnésium et de luciférase qui catalyse la réaction suivante :

$$ATP + D\text{-luciférine} + O2 \xrightarrow[\text{Mg}^{++}]{\text{luciférase}} \text{Oxyluciférase} + Ppi + AMP + CO2 + \text{lumière}$$

Le rendement de cette réaction, correspondant au rapport du nombre de photons émis sur le nombre de moles d'ATP ayant réagi, est total.

Les cellules sont cultivées en plaques 24 puits en présence ou en absence du produit, du polluant, ou du mélange produit + polluant. Les mesures sont réalisées en triplicata. Les tapis cellulaires sont lavés en PBS et les cellules sont lysées sur glace par 100 $\mu$L d'eau contenant 0,2 % de Triton X100 (Sigma). Le dosage d'ATP est réalisé avec un prêt-à-monter de dosage "ATP bioluminescence kit HS II" (Roche Diagnostics GmbH). Les résultats sont obtenus à partir d'une gamme d'étalonnage.

Effet de l'ajout de produit dans le milieu de culture non pollué sur le dosage de l'ATP

| Produit ajouté dans le milieu | Quantité d'ATP mesurée (en nmole/mL) | % par rapport au témoin négatif |
|---|---|---|
| 0 (témoin) | 3,152 | 100 |
| Solution à 6,4 $\mu$g/mL de produit bactérien | 3,091 | 98 |

Le produit bactérien testé n'a pas d'effet significatif sur la quantité d'ATP synthétisée. L'inhibition due au produit est négligeable.

Effet de la pollution du milieu de culture sur le dosage de l'ATP

| Type de milieu | Quantité d'ATP mesurée (en nMole/mL) | % par rapport au témoin négatif (milieu non pollué) |
|---|---|---|
| Milieu non pollué | 3,152 | 100 |
| Milieu pollué dilué au 1/2 | 2,373 | 75 |
| Milieu pollué non dilué | 1,995 | 63 |

Le polluant pur ou dilué au demi inhibe la synthèse d'ATP.

Dosage de l'ATP synthétisé en présence du produit bactérien et de milieu pollué

L'indice de protection est calculé de la manière suivante :

$$I.P.= 100 - \{100 \text{ x } (\{\text{milieu non pollué traité} - \text{milieu pollué traité}\} \div \{\text{milieu non pollué témoin} - \text{milieu pollué témoin}\})\}$$

| Milieu de culture | Produit ajouté | Quantité d'ATP nmole/mL | % du témoin | I.P. |
|---|---|---|---|---|
| Milieu non pollué | | 2,206 | 100 | - |
| Milieu pollué dilué au 1/2 | Solution à 6,4 $\mu$g/mL de produit | 2,162 | 98 | 92 |
| Milieu pollué non dilué | | 2,118 | 96 | 88 |

(suite)

| Milieu de culture | Produit ajouté | Quantité d'ATP nmole/mL | % du témoin | I.P. |
|---|---|---|---|---|
| Milieu non pollué | | 2,309 | 100 | - |
| Milieu pollué dilué au 1/2 | Témoin sans produit | 1,767 | 77 | - |
| Milieu pollué non dilué | | 1,576 | 68 | - |

D'après ces résultats, le produit bactérien testé ne réduit pas le stock d'ATP dans le milieu non pollué et protège les cellules en culture de l'effet du polluant (respectivement 88 et 92 % en indice de protection).

**Exemple 5 :** Détermination *in vitro* de l'effet anti-radicalaire du produit à tester sur une suspension de kératinocytes humains.

Des kératinocytes humains sont cultivés dans du milieu KGM (Kératinocytes Growth Medium) enrichi en facteurs de croissance et antibiotiques. Ce milieu est en fait composé de tampon HEPES à pH 7,4, d'acides aminés essentiels et non essentiels, de vitamines et minéraux ainsi que de composés organiques et de sels inorganiques. Les facteurs de croissance sont du HKGS (Human Keratinocyte Growth Supplement), du BPE (Bovine Pituitary Extract), de l'insuline bovine, de l'hydrocortisone; de la transferrine bovine et de l'EGF humain (Epidermal Growth Factor).

Les cellules sont trypsinées et mises en suspension à $10^6$ cellules par millilitre de milieu. Ces kératinocytes sont irradiés pendant 30 minutes avec une lampe Hélarium émettant des ultraviolets A et B afin d'activer la synthèse des radicaux libres. La suspension de kératinocytes, contenant ou non le produit à tester, est acidifiée à la fin de l'irradiation (50 $\mu$l d'acide citrique à 2 moles/L pour 260 $\mu$l de suspension cellulaire de façon à obtenir une suspension à pH 3,3) et une solution de catalase est ajoutée. En fait, 100 $\mu$l de méthanol/terbutanol et 400 $\mu$l d'eau distillée sont ajoutées à la suspension cellulaire puis 15 $\mu$l de catalase à 0,2 mg/mL pour 100 $\mu$l de la suspension précédente sont ajoutés afin d'éliminer le peroxyde d'hydrogène pouvant réagir et créer un faux signal.

Un réactif luminescent est alors ajouté. Les peroxydes formés, résultant de l'action des radicaux libres sur les cellules, sont ensuite dosés par chemiluminescence. La lecture effectuée pendant une minute par le luminomètre totalise le nombre d'unités relatives de luminescence (URL) émises.

Le produit à tester est dilué dans du milieu de culture à une concentration de 6,4 $\mu$g/mL et ajouté ou non à la suspension de kératinocytes.

Le pouvoir de protection du produit contre la synthèse des radicaux libres est représenté par l'efficacité E exprimée en % et calculée par la formule :

$$E\% = \{(CI\text{-}CTI) \div (CI\text{-}CNI)\} \times 100$$

où CI représente les cellules irradiées non traitées par le produit, CTI les cellules irradiées et traitées avec les produit et CNI les cellules non irradiées et non traitées.

Estimation de l'action du produit bactérien sur la synthèse de radicaux libres

| | Témoin Cellules non irradiées (CNI) | Témoin Cellules irradiées (CI) | Cellules non irradiées avec produit (CT) | Cellules irradiées avec produit (CTI) |
|---|---|---|---|---|
| Essai 1 | 1211 | 68222 | 7283 | 37678 |
| Essai 2 | 1800 | 101000 | 7300 | 17100 |
| Essai 3 | 1281 | 104458 | 3444 | 46844 |
| Moyenne | 1431 | 91227 | 6009 | 33874 |
| SEM | 186 | 11545 | 1283 | 8795 |
| E % | **64 %** | | | |

Dans les conditions de cette étude, l'ajout de produit bactérien à des cellules exposées à des ultraviolets les protège de la formation de radicaux libres. Ce produit présente un effet protecteur anti-radicalaire de 64 %.

**Exemple 6 : Quantification *in vivo* de l'effet anti-pollution d'une crème contenant le produit bactérien**

Il s'agit d'une étude comparative (produit contre placebo) randomisée et réalisée en intra-individuel. Les deux crèmes sont appliquées sur chaque volontaire.

Les crèmes cosmétiques sont réalisées selon la formule suivante :

|  | Crème 5090 En % | Crème 5091 En % |
|---|---|---|
| Huile végétale | 8,00 | 8,00 |
| Cyclométhicone | 6,70 | 6,70 |
| Stéarate de glycol auto-émulsionnable | 5,80 | 5,80 |
| Huile minérale | 4,00 | 4,00 |
| Alcool céthylique | 1,00 | 1,00 |
| Conservateur | 0,50 | 0,50 |
| Carbomer | 0,40 | 0,40 |
| Triéthalolamine | 0,40 | 0,40 |
| Extrait aqueux *d'Halobacterium halobium* obtenu selon le procédé de l'exemple 1 à 1mg/g de crème | - | 0,10 |
| Eau déminéralisée | Q.S.P. 100 % | Q.S.P. 100 % |

Le pouvoir anti-pollution d'un produit cosmétique ou dermopharmaceutique est évalué en calculant le pourcentage de protection de la peau vis-à-vis de microparticules de carbone par rapport à une zone non traitée (et/ou à un placebo). Les particules polluantes utilisées comme marqueurs de la pollution atmosphérique sont des microparticules de carbone en suspension dans l'eau.

Le protocole d'inclusion des volontaires dans cette étude est défini par les points suivants :

■ Sexe féminin
■ Majeure
■ Type caucasien
■ Phototype II-III-IV

Sont exclues de ce protocole les femmes enceintes ou allaitant, les femmes présentant une pathologie cutanée ou une desquamation et/ou un érythème sur la zone d'expérience ainsi que les volontaires atteintes d'une maladie grave ou évolutive. Les femmes sous médication d'anti-inflammatoires, corticoïdes ou rétinoïdes sont également exclues de ce protocole.

Ce protocole est réalisé sur dix volontaires qui ont accepté de ne pas utiliser de produits dermopharmaceutiques ou cosmétiques au niveau des zones à étudier le jour de l'étude.

Tableau récapitulatif des volontaires incluses dans le protocole

| Nombre | Sexe | Age moyen | Type | Phototype |
|---|---|---|---|---|
| 10 | Féminin | 34 ± 4 ans | Caucasien | III |

Deux types de crèmes sont testés :

■ Crème 5090 qui est un placebo
■ Crème 5091 qui a une composition identique à celle de la crème 5090 dans laquelle on a ajouté le produit bactérien à une concentration finale de 1mg/mL)

Une zone de 16 cm$^2$ est définie sur chaque avant-bras (une zone placebo et une zone traitée). A t=0, on applique le placebo et le produit à tester sur les deux zones définies en quantité standardisée (2 $\mu$L/cm$^2$) c'est-à-dire que l'on applique sur chaque zone 32 $\mu$L de crème 5090 ou 5091 selon la zone, puis on masse légèrement et de façon circulaire à l'aide d'un doigtier pendant 15 secondes la zone concernée. A t=20min, on applique le polluant sur chaque zone définie en quantité standardisée (2 $\mu$L/cm$^2$). A t=60min, après rinçage et séchage standardisés des zones étudiées, une acquisition d'image de chaque zone est prise (trois prises d'images par zone). La visualisation des particules polluantes à la surface de la peau est effectuée à l'aide d'un vidéomicroscope muni d'un objectif x100, mobile et à fibre optique, couplé à un système informatique d'acquisition d'image.

Afin d'exprimer la diminution de "pollution" observée sur la peau de la zone traitée par le produit ou le placebo, les résultats sont donnés en pourcentage de protection (P %) selon la formule suivante :

$$P \% = \{(ZAV\text{-}ZAP) \div ZAV\} x 100$$

où ZAV est la quantité de particules de carbone mesurée (en pixels) sur la zone avant rinçage et ZAP la quantité de particules de carbone mesurée (en pixels) après rinçage. SEM représente l'écart-type sur la moyenne des résultats des dix volontaires.

Si P % =100 % ; alors la protection contre la pollution est totale, la surface de la peau ne présente plus aucune trace de particules de carbone.

Taux de particules polluantes sur les zones étudiées avant et après rinçage

| | Crème 5090 (placebo) | | Crème 5091 (5090 + produit bactérien à 1mg/mL) | |
|---|---|---|---|---|
| | Avant rinçage (ZAV) | Après rinçage (ZAP) | Avant rinçage (ZAV) | Après rinçage (ZAP) |
| Moyenne des volontaires | 71327 | 31725 | 65196 | 14792 |
| SEM | 6706 | 4557 | 5508 | 2796 |
| P % | 56 | | 77 | |

Dans les conditions de cette étude, on note une différence entre les résultats obtenus avec le placebo et ceux obtenus par la crème contenant le produit à tester. Le placebo protège la peau de 56 % alors que la crème contenant l'extrait bactérien protège la peau de 77 %.

### Exemple 7 : Types de formulation cosmétique

Formulation de lotion protectrice hydratante

| | |
|---|---|
| Eau déminéralisée | Q.S.P. 100mL |
| Glycerin | 5,00 |
| PEG-40 Hydrogénated Castor Oil | 3,00 |
| Panthenol | 0,30 |
| Chlorhexidine | 0,20 |
| Extrait d'*Halobacterium halobium* selon l'invention | 0,02 |
| Parfum | Q.S. |

Formulation de lait solaire

| Eau déminéralisée | Q.S.P. 100mL |
|---|---|
| Mineral oil | 7,00 |
| Glycol stéarase S.E. | 6,20 |
| Isopropyl myristate | 5,50 |
| Ethylhexyl Methoxycinnamate | 4,00 |
| Butyl Methoxidibenzoylmethane | 2,00 |
| Cetyl alcohol | 0,30 |
| Carbomer | 0,30 |
| Triethanolamine | 0,25 |
| Extrait d'*Halobacterium halobium* selon l'invention | 0,10 |
| Imidazolidinyl Urea | 0,20 |
| Methylbaraben | 0,05 |
| Propylparaben | 0,05 |
| Parfum | Q.S. |

Formulation d'un gel base de maquillage

| Eau déminéralisée | Q.S.P. 100 mL |
|---|---|
| PEG-7 Glyceryl Cocoate | 4,50 |
| Sorbitol | 3,00 |
| Propylene Glycol | 2,50 |
| Carbomer | 0,60 |
| Triethanolamine | 0,60 |
| Polyvinyl pyrrolidone | 0,50 |
| Extrait d'*Halobacterium halobium* selon l'invention | 0,10 |
| Imidazolidinyl Urea | 0,40 |
| Parfum | Q.S. |

Formulation d'une crème cosmétique base de démaquillage

| Eau déminéralisée | Q.S.P. 100 mL |
|---|---|
| Cetearyl Glucoside | 6,30 |
| Cyclomethicone | 7,00 |
| Isostearyl Isostearate | 6,00 |
| Cetearyl alcohol | 3,00 |
| Cetyl alcohol | 1,50 |

(suite)

| Eau déminéralisée | Q.S.P. 100 mL |
|---|---|
| Butyrospermum Parkii (shea butter) fruit | 2,00 |
| PVP/Dimethylaminoethyl Methacrylate copolymer | 0,50 |
| Dimethicone | 0,50 |
| Xanthan gum | 0,30 |
| Extrait d'*Halobacterium halobium* selon l'invention | 0,05 |
| Imidazolidinyl Urea | 0,20 |
| Parfum | Q.S. |

## Revendications

**1.** Utilisation en cosmétique d'un produit comprenant une fraction glycoprotéique d'archaebactéries, à l'exclusion de toute utilisation ayant un effet thérapeutique.

**2.** Utilisation selon la revendication 1 **caractérisée par le fait que** le produit comprend, en poids, de 25 à 40 % de glycoprotéines d'archaebactéries.

**3.** Utilisation selon l'une des revendications 1 ou 2 **caractérisée par le fait que** l'archaebactérie est une halobactérie.

**4.** Utilisation selon l'une quelconque des revendications 1 à 3 **caractérisée par le fait que** la halobactérie est *Halobacterium halobium*.

**5.** Utilisation selon l'une quelconque des revendications 1 à 4 pour la protection de la peau vis-à-vis de la pollution par les gaz d'échappement.

**6.** Composition cosmétique **caractérisée par le fait qu'**elle contient, dans un milieu cosmétiquement acceptable, au moins un produit comprenant une fraction glycoprotéique d'archaebactéries et qu'elle se présente sous la forme d'un gel, d'un lait, d'une lotion ou d'une crème.

**7.** Utilisation en cosmétique d'une composition selon la revendication 6, à l'exclusion de toute utilisation ayant un effet thérapeutique.

**8.** Utilisation d'un produit comprenant une fraction glycoprotéique d'archaebactéries pour la préparation d'une composition destinée à la protection de la peau vis-à-vis des rayonnements ultraviolets.

## Claims

**1.** Cosmetic use of a product comprising a glycoprotein fraction from archaebacteria, excluding any use having a therapeutic effect.

**2.** Use according to Claim 1, **characterized in that** the product comprises, by weight, from 25 to 40% of glycoproteins from archaebacteria.

**3.** Use according to either of Claims 1 and 2, **characterized in that** the archaebacterium is a halobacterium.

**4.** Use according to any one of Claims 1 to 3, **characterized in that** the halobacterium is *Halobacterium halobium*.

**5.** Use according to any one of Claims 1 to 4, for protecting the skin against pollution caused by exhaust gases.

**6.** Cosmetic composition, **characterized in that** it contains, in a cosmetically acceptable medium, at least one product comprising a glycoprotein fraction from archaebacteria and that it is provided in the form of a gel, a milk, a lotion, or a cream.

7. Cosmetic use of a composition according to Claim 6, excluding any use having a therapeutic effect.

8. Use of a product comprising a glycoprotein fraction from archaebacteria, for the preparation of a composition intended for protecting the skin against ultraviolet radiation.


**Patentansprüche**

1. Kosmetische Verwendung eines Produkts, das eine Archäbakterien-Glykoproteinfraktion enthält, mit Ausnahme jeglicher Verwendung, die eine therapeutische Wirkung beinhaltet.

2. Verwendung nach Anspruch 1, **dadurch gekennzeichnet, daß** das Produkt 25 bis 40 Gew.-% Archäbakterien-Glykoproteine enthält.

3. Verwendung nach Anspruch 1 oder 2, **dadurch gekennzeichnet, daß** es sich bei dem Archäbakterium um ein Halobakterium handelt.

4. Verwendung nach einem der Ansprüche 1 bis 3, **dadurch gekennzeichnet, daß** es sich bei dem Halobakterium um *Halobacterium halobium* handelt.

5. Verwendung nach einem der Ansprüche 1 bis 4 zum Schutz der Haut gegen Umweltverschmutzung durch Auspuffgase.

6. Kosmetische Zusammensetzung, **dadurch gekennzeichnet, daß** sie in einem kosmetisch unbedenklichen Medium mindestens ein Produkt mit einer Archäbakterien-Glykoproteinfraktion umfaßt und daß sie in Form eines Gels, einer Milch, einer Lotion oder einer Creme vorliegt.

7. Kosmetische Verwendung einer Zusammensetzung nach Anspruch 6 mit Ausnahme jeglicher Verwendung, die eine therapeutische Wirkung beinhaltet.

8. Verwendung eines Produkts, das eine Archäbakterien-Glykoproteinfraktion umfaßt, zur Herstellung einer Zusammensetzung zum Schutz der Haut gegen Ultraviolettstrahlen.